# EUROPÄISCHE PATENTSCHRIFT

(11) **EP 1 483 250 B1**
(45) Veröffentlichungstag und Bekanntmachung des Hinweises auf die Patenterteilung: **28.04.2010**
(21) Anmeldenummer: 03743330.7
(22) Anmeldetag: 26.02.2003
(51) Int. Cl.: C07D 251/54

(54) **VERFAHREN ZUR HERSTELLUNG EINER TAUTOMEREN FORM (I) VON 2, 4, 6-TRIANILINO-P- (CARBO-2 -ETHYLHEXYL-1 -OXY)-1, 3, 5-TRIAZIN**
METHOD FOR PRODUCING THE TAUTOMERIC FORM (I) OF 2, 4, 6-TRIANILINO-P- (CARBO-2'-ETHYLHEXYL-1'-OXY)-1, 3, 5-TRIAZINE
PROCEDE DE PRODUCTION DE LA FORME TAUTOMERE (I) DE 2, 4, 6-TRIANILINO-P-(CARBO-2'-ETHYLHEXYL-1'-OXY)-1, 3, 5-TRIAZINE

(30) Priorität: 01.03.2002 DE 10208840
(43) Veröffentlichungstag der Anmeldung: 08.12.2004
(73) Patentinhaber: BASF SE, 67056 Ludwigshafen (DE)
(72) Erfinder: GÜMBEL, Helmut, 67814 Dannenfels (DE); KNEUPER, Heinz-Josef, 67150 Niederkirchen (DE); BECKER, Rainer, 67098 Bad Dürkheim (DE); BERTLEIN, Gerhard, 69151 Neckargemünd (DE); DRÖGEMÜLLER, Michael, 68167 Mannheim (DE)
(86) Internationale Anmeldenummer: PCT/EP2003/001931
(87) Internationale Veröffentlichungsnummer: WO 2003/074499

(56) Entgegenhaltungen:
- EP-A- 0 087 098
- EP-A- 0 202 611

## Beschreibung

Die vorliegende Erfindung betrifft ein neues Verfahren zur Herstellung der tautomeren Form I von 2,4,6-Trianilino-p-(carbo-2'-ethylhexyl-1'-oxy)-1,3,5-triazin 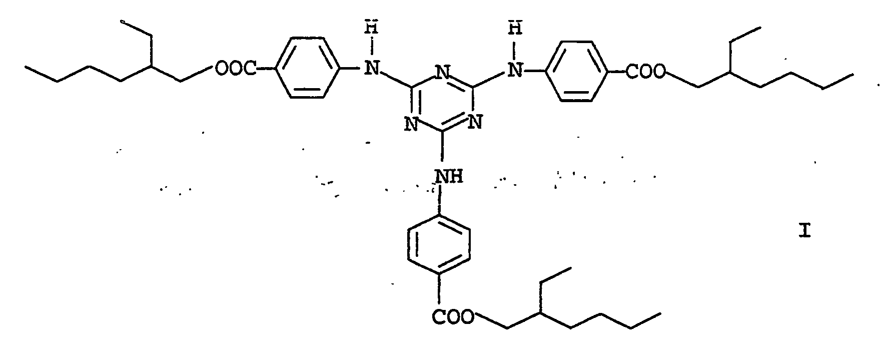 durch Kristallisation aus einem Tautomerengemisch.

Außerdem betrifft die Erfindung die Verwendung der so isolierten Form I als Lichtschutzmittel in kosmetischen Zubereitungen.

In der EP-B 087 098 (1) werden die Herstellung von 2,4,6-Trianilino-p-(carbo-2'-ethylhexyl-1'-oxy)-1,3,5-triazin aus Cyanurchlorid und p-Aminobenzoesäure-2-ethylhexylester und die Reinigung des Rohproduktes durch Umkristallisation aus Benzin beschrieben.

Die DE-A 35 18 670 (2) betrifft die Verwendung von Estern aus einer verzweigten Alkansäure mit 6 bis 10 C-Atomen und einem gesättigten aliphatischen Alkohol mit 10 bis 20 C-Atomen als Lösungsmittel bei der Herstellung von s-Triazinderivaten wie 2,4,6-Trianilino-p-(carbo-2'-ethylhexyl-1'oxy)-1,3,5-triazin.

Die bei den genannten Verfahren anfallenden Produkte stellen in der Regel Tautomerengemische dar, wie sich leicht aus den zugehörigen IR-Spektren ableiten läßt. Es ist beispielsweise aus J. Elguero, C. Marzin, A.R. Katritzky und P. Linda, Advances in Heterocyclic Chemistry, Supplement 1, The Tautomerism of Heterocycles, Academic Press New York, 1976, Kp. 2, S. 168 bekannt, daß bei Amino-s-triazin-Derivaten allgemein Tautomere bezüglich der NH-Protonen auftreten können.

Setzt man die so erhaltenen 2,4,6-Trianilino-p-(carbo-2'-ethylhexyl-1'-oxy)-1,3,5-triazin-Tautomerengemische in kosmetischen Zubereitungen ein, tritt häufig das Problem des allmählichen Wiederausfallens von in den verwendeten kosmetischen Ölen in Lösung gegangener Substanz bei der Lagerung auf.

Weiterhin ist für die Anwender wichtig, daß der Stoff bei der Prüfung auf seine Eignung für die Anwendung und bei der Anwendung selbst chemisch einheitlich ist. Insbesondere für toxikologische Untersuchungen und Verträglichkeitstests, deren Ergebnisse für Inhaltsstoffe kosmetischer Zubereitungen naturgemäß besonders relevant sind, sind Gemische von Stoffen unterschiedlicher physikalischer Eigenschaften, also auch Tautomerengemische, nicht verwendbar, weil bei Verfütterungsversuchen mit ihnen an lebende Organismen in diesen Prüforganismen keine konstante Zusammensetzung vorausgesetzt werden kann, so daß das Ergebnis dieser Tests dadurch verfälscht werden könnte. Außerdem unterliegen derartige Gemische schon aufgrund geringfügiger Änderungen der Herstellungsbedingungen oft starken Schwankungen in der Zusammensetzung, so daß eine bestimmte Zusammensetzung nur schwer reproduzierbar ist.

Der vorliegenden Erfindung lag daher die Aufgabe zugrunde, den geschilderten Nachteilen bei der Anwendung von 2,4,6-Trianilino-p-(carbo-2'ethylhexyl-1'-oxy)-1,3,5-triazin abzuhelfen.

Diese Aufgabe wurde gelöst durch ein Verfahren zur Herstellung der tautomeren Form I von 2,4,6-Trianilino-p-(carbo-2'-ethylhexyl-1'-oxy)-1,3,5-triazin durch Kristallisation aus einem Tautomerengemisch in Gegenwart eines oder mehrerer Lösungsmittel, ausgewählt aus der Gruppe, bestehend aus aliphatischen Alkoholen mit 2 bis 8 C-Atomen, aliphatischen Carbonsäurealkylestern mit insgesamt 3 bis 10 C-Atomen, aromatischen Carbonsäurealkylestern mit insgesamt 8 bis 12 C-Atomen, aliphatischen Kohlensäureestern mit insgesamt 3 bis 9 C-Atomen, Carbonsäurenitrilen mit insgesamt 2 bis 8 C-Atomen, Dialkylketonen mit insgesamt 3 bis 6 C-Atomen und aliphatischen Sulfonen mit insgesamt 3 bis 6 C-Atomen, wobei das Lösungsmittel oder die Lösungsmittelmischung zusätzlich noch bis zu 30 Gew.-% eines Kohlenwasserstoffs enthalten kann.

Als aliphatische Alkohole mit 2 bis 8 C-Atomen kommen sowohl gradkettige, verzweigte als auch cyclische Alkohole in Betracht. Beispielsweise zu nennen sind Ethanol, n-Propanol, iso-Propanol, n-Butanol, tert.-Butanol, n-Pentanol, n-Hexanol, n-Octanol, 2-Ethylhexanol und Cyclohexanol. Ein bevorzugt verwendeter Alkohol als Lösungsmittel ist Ethanol.

Als aliphatische Carbonsäurealkylester mit insgesamt 3 bis 10 C-Atomen eignen sich beispielsweise gradkettige Carbonsäureester wie Ameisensäureethylester, Ameisensäure-n-propylester, Essigsäuremethylester, Essigsäureethylester, Essigsäure-n-propylester, Essigsäure-n-butylester, Buttersäuremethylester, Buttersäureethylester, Buttersäure-n-propylester und Buttersäure-n-butylester. Bevorzugt sind aliphatische Carbonsäurealkylester mit insgesamt 3 bis 6 C-Atomen, besonders bevorzugt Essigsäureethylester.

Unter aromatische Carbonsäurealkylester mit insgesamt 8 bis 12 C-Atomen sind beispielsweise Benzoesäuremethylester, Benzoesäureethylester, Benzoesäure-n-propylester, Benzoesäure-iso-propylester, Benzoesäure-n-butylester, Benzoesäure-tert.-butylester, bevorzugt Benzoesäuremethylester zu verstehen.

Als aliphatische Kohlensäureester mit insgesamt 3 bis 9 C-Atomen sind Dimethylcarbonat, Diethylcarbonat, Di-n-propylcarbonat, Di-iso-propylcarbonat, Di-n-butylcarbonat und Propylencarbonat, bevorzugt Dimethylcarbonat zu nennen.

Als Carbonsäurenitrile mit insgesamt 2 bis 8 C-Atomen eignen sich beispielsweise Acetonitril, Propiononitril, n-Butyronitril, Benzonitril, bevorzugt Acetonitril.

Als Dialkylketone mit insgesamt 3 bis 6 C-Atomen kommen u.a. gradkettige, verzweigte sowie cyclische Dialkylketone, beispielsweise Aceton, Butanon, 2-Pentanon, 3-Pentanon, 2-Hexanon, 3-Hexanon und Cyclohexanon, bevorzugt Aceton in Betracht.

Als ein Vertreter aliphatischer Sulfone mit insgesamt 3 bis 6 C-Atomen ist beispielsweise Tetramethylensulfon zu nennen.

Die gegebenenfalls als Cosolvens mitverwendeten Kohlenwasserstoffe können sowohl aliphatische Kohlenwasserstoffe.wie Pentan, Hexan, Petrolether, Ligroin oder Cyclohexan und/oder aromatische Kohlenwasserstoffe wie Toluol oder Xylol sein. Diese Kohlenwasserstoffe können in einer Menge bis zu 30 Gew.-%, vorzugsweise in einer Menge von nicht mehr als 10 Gew.-%, mitenthalten sein.

Eine bevorzugte Ausführungsform des erfindungsgemäßen Verfahrens ist **dadurch gekennzeichnet, daß** man die Kristallisation in einem der eingangs genannten aliphatischen Alkoholen mit 2 bis 4 C-Atomen, in einem der eingangs genannten aliphatischen Carbonsäurealkylestern mit insgesamt 3 bis 6 C-Atomen oder einer Mischung hieraus durchführt.

Besonders bevorzugt wird die Kristallisation in Ethanol, Essigsäureethylester oder einer Mischung hieraus als Lösungsmittel durchführt.

Die im Rahmen der vorliegenden Erfindung verwendeten Lösungsmittel können sowohl wasserfrei sein als auch einen Wassergehalt von maximal 10 Gew.-%, bevorzugt von maximal 5 Gew.-%, besonders bevorzugt von maximal 2 Gew.-% aufweisen. Dies gilt insbesondere für die oben genannten aliphatischen Alkohole sowie für die aliphatischen Carbonsäurealkylester.

Die Umkristallisation erfolgt in der Regel durch Auflösen des Tautomerengemisches im Lösungsmittel oder der Lösungsmittelmischung bei Temperaturen von 40 bis 120°C, vorzugsweise 60 bis 100°C. Die Menge an Lösungsmittel beträgt zweckmäßigerweise 100 bis 2000 Gew.-%, vorzugsweise 150 bis 600 Gew.-%, bezogen auf das Tautomerengemisch. Nach Abfiltrieren von unlöslichen Anteilen in der Hitze, wobei sich die Mitverwendung von Filtrierhilfsmitteln empfiehlt, wird bei Temperaturen von etwa 15 bis 40°C die tautomere Form I durch Kristallisation abgeschieden. Da I zur Bildung übersättigter Lösungen neigt, ist die Zugabe von Impfkristallen bei dieser Temperatur zweckmäßig. Das Abfiltrieren, Trocknen und gegebenenfalls Konfektionieren erfolgt nach den üblichen Methoden.

Das eingesetzte Tautomerengemisch wird zweckmäßigerweise direkt als Schmelze mit dem genannten Lösungsmittel oder der Lösungsmittelmischung versetzt. Man kann aber auch das Tautomerengemisch in kristallisierter Form in das heiße Lösungsmittel eintragen oder es zusammen mit diesem erhitzen.

Die tautomere Form I ist als solche stabil und wandelt sich in fester und in gelöster Form nicht in das Tautomerengemisch zurück.

Gegenstand der vorliegenden Erfindung ist außerdem die Verwendung der so isolierten tautomeren Form I als Lichtschutzmittel in kosmetischen Zubereitungen. Beispiele für die Zusammensetzungen derartiger kosmetischer Zubereitungen wie Lichtschutzemulsionen, Ölin-Wasser-Lichtschutzcremes, Wasser-in-Öl-Lichtschutzcremes oder Lichtschutzschäumen finden sich in der Patentschrift (1).

Als Lösungsmittel für I dienende übliche kosmetische Öle sind neben den der Patentveröffentlichung (2) zugrundeliegenden C₆-C₁₀-Alkansäure-C₁₀-C₂₀-alkylestern wie 2-Ethylhexansäure-cetylstearylester beispielsweise Erdnussöl, Olivenöl, Isopropylstearat, Isopropylmyristat, Kokosnußfettsäure-Triglyceride, Capryl-caprinsäuretriglycerid, Triethylcitrat, Polyethylenglykol-glycerylcocoat, Diisopropyladipat, propoxylierter Myristylalkohol oder Gemische hiervon. Die Löslichkeiten der tautomeren Form I ist in den genannten kosmetischen Ölen ausreichend hoch. Die Lagerbeständigkeit dieser Lösungen bezüglich Trübungen und Ausfällungen ist deutlich besser, weil diese Lösungen über eine längere Zeit klar bleiben.

Ein weiterer Vorteil des erfindungsgemäßen Verfahrens ist die gute Kristallisierbarkeit und, damit verbunden, die gute Filtrierbarkeit und das gute Trocknungsverhalten des tautomerenreinen Produktes.

### Beispiel 1

### Herstellung der tautomeren Form I durch Umkristallisation aus Ethanol

250 kg s-Trichlortriazin (entsprechend 1,36 kmol) wurden mit 1025 kg p-Aminobenzoesäure-2-ethylhexylester (entsprechend 4,11 kmol) in Xylol als Lösungsmittel zum 2,4,6-Trianilino-p-(carbo-2'-ethylhexyl-1'-oxy)-1,3,5-triazin-Tautomerengemisch umgesetzt.

Nach Abdestillieren des Lösungsmittels wurde die 115°C heiße Schmelze innerhalb von 5 bis 6 Stunden mit 4500 kg Ethanol (entsprechend 400 Gew.-%, bezogen auf das eingesetzte Tautomerengemisch) versetzt, wobei die Temperatur zwischen 65 und 80°C gehalten wurde. Nach Zugabe von 25 kg eines üblichen Filtrierhilfsmittels (Celite^{®}) wurden bei 65 bis 70°C unlösliche Anteile abfiltriert und die Lösung wurde auf 20°C abgekühlt. Danach wurde durch Animpfen mit 150 kg der tautomeren Form I (grobkristallines, nicht getrocknetes Produkt aus einem früheren Ansatz entsprechend 110 kg trockenem Produkt) die Kristallisation eingeleitet. Nach 18 Stunden Rühren bei 20°C wurde das Produkt über eine Zentrifuge ausgetragen, mit Ethanol gewaschen und bei 60°C/20 mbar getrocknet. Es resultierten 915 kg tautomerenreines I (72 % Ausbeute, bezogen auf eingesetztes s-Trichlortriazin und nach Abzug der Impfkristallmenge).

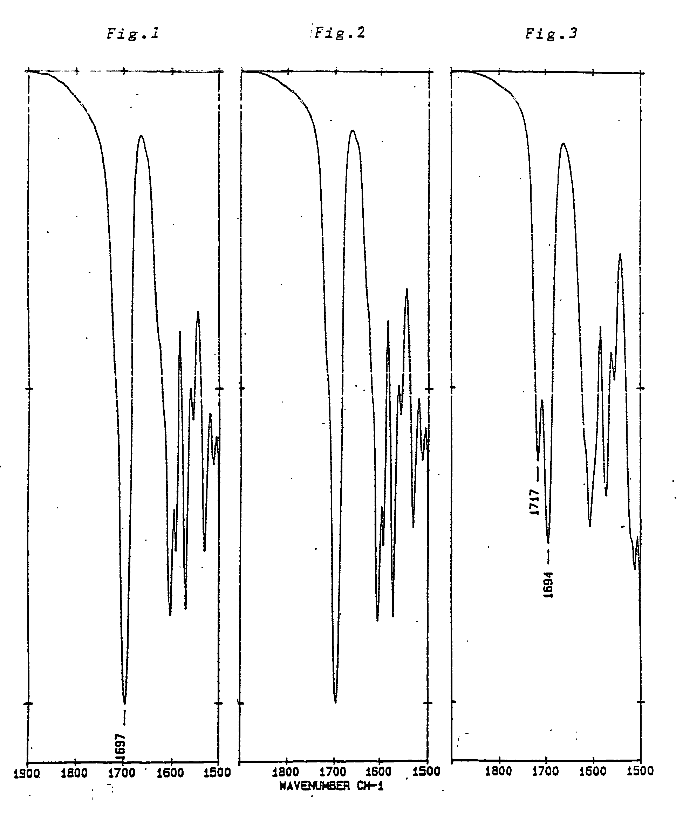

Das IR-Spektrum des Produktes (KBr-Pressling) zeigte im Bereich der Carbonyl-Streckschwingungen nur eine Bande für die Carboxylgruppen bei 1697 cm-1 (siehe Figur 1) im Gegensatz zu der entsprechenden Doppelbande des Produktes aus Vergleichsbeispiel A.

### Beispiel 2

Herstellung der tautomeren Form I durch Umkristallisation aus Essigsäureethylester

Analog zu Beispiel 1 wurde die Umkristallisation mit 400 Gew.-% Essigsäureethylester, bezogen auf das eingesetzte Tautomerengemisch, durchgeführt. Das tautomerenreine Produkt wurde in einer Ausbeute von 58 % erhalten.

Das IR-Spektrum des Produktes (KBr-Pressling) zeigte im Bereich der Carbonyl-Streckschwingungen nur eine Bande für die Carboxylgruppen bei 1697 cm-1 (siehe Figur 2) im Gegensatz zu der entsprechenden Doppelbande des Produktes aus Vergleichsbeispiel A.

### Beispiele 3 bis 12

Analog zu Beispiel 1 wurde die Umkristallisation jeweils mit Ethylhexanol (Bsp. 3), Cyclohexanol (Bsp. 4), Benzoesäuremethylester (Bsp. 5), Tetramethylensulfon (Bsp. 6), Dimethylcarbonat (Bsp. 7), Methylethylketon (Bsp. 8), Acetonitril (Bsp. 9) und Aceton (Bsp. 10) sowie mit Lösungsmittelgemischen aus 90 Gew.-% Ethanol/10 Gew.-% Xylol (Beispiel 11) und 90 Gew.-% Ethylacetat/10 gew.-% Xylol (Beispiel 12) durchgerührt.

Die IR-Spektren der jeweils isolierten Produkte (KBr-Pressling) zeigten im Bereich der Carbonyl-Streckschwingungen ebenfalls nur eine Bande für die Carboxylgruppen bei 1697 cm-1.

### Vergleichsbeispiel A

### Umkristallisation aus Benzin

Gemäß der Patentschrift (1) wurde das bei der Herstellung angefallene Tautomerengemisch aus Benzin der Siedegrenzen 120 bis 130°C umkristallisiert.

Das IR-Spektrum des Produktes (KBr-Pressling) zeigt im Bereich der Carbonyl-Streckschwingungen eine Doppelbande für die Carbonylgruppen bei 1717/1694 cm⁻¹ (siehe Figur 3), was als Beweis für das Vorliegen von zwei tautomeren Formen anzusehen ist. Das IR-Spektrum des Tautomerengemisches vor der Umkristallisation war im wesentlichen identisch mit diesem Spektrum.

### Beispiel 13

Lagerbeständigkeit von Lösungen der tautomeren Form I in kosmetischen ölen

Die tautomerenreine Verbindung I wurde jeweils in C₈-C₁₀-Kokosnussfettsäure-Triglycerid (Löslichkeit 5,8 g pro 100 g öl) und 2-Ethylhexansäure-cetylstearylester (Löslichkeit 1,0 g pro 100 g öl) gelöst. Beide Lösungen waren nach 2 Tagen immer noch klar.

Dagegen wurden entsprechende anfangs klare Lösungen des Tautomerengemisches mit einem in weiten Grenzen variierenden Gehalt an I schon nach 1 Tag trübe und ließen nach 2 Tagen bereits Ausfällungen erkennen.

## Patentansprüche

1. Verfahren zur Herstellung der tautomeren Form I von 2,4,6-Trianilino-p-(carbo-2'-ethylhexyl-1'-oxy)-1,3,5-triazin 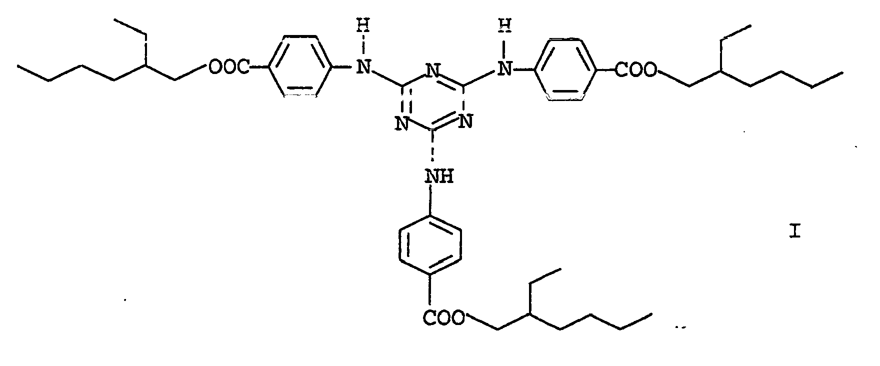 durch Kristallisation aus einem Tautomerengemisch in Gegenwart eines oder mehrerer Lösungsmittel, ausgewählt aus der Gruppe, bestehend aus aliphatischen Alkoholen mit 2 bis 8 C-Atomen, aliphatischen Carbonsäurealkylestern mit insgesamt 3 bis 10 C-Atomen, aromatischen Carbonsäurealkylestern mit insgesamt 8 bis 12 C-Atomen, aliphatischen Kohlensäureestern mit insgesamt 3 bis 9 C-Atomen, Carbonsäurenitrilen mit insgesamt 2 bis 8 C-Atomen, Dialkylketonen mit insgesamt 3 bis 6 C-Atomen und aliphatischen Sulfonen mit insgesamt 3 bis 6 C-Atomen, wobei das Lösungsmittel oder die Lösungsmittelmischung zusätzlich noch bis zu 30 Gew.-% eines Kohlenwasserstoffs enthalten kann.

2. Verfahren nach Anspruch 1, **dadurch gekennzeichnet, daß** man die Kristallisation in einem aliphatischen Alkohol mit 2 bis 4 C-Atomen, in einem aliphatischen Carbonsäurealkylester mit insgesamt 3 bis 6 C-Atomen oder einer Mischung hieraus durchführt.

3. Verfahren nach einem der Ansprüche 1 oder 2, **dadurch gekennzeichnet, daß** man die Kristallisation in Ethanol, Essigsäureethylester oder einer Mischung hieraus durchführt.

4. Verwendung der gemäß einem der Ansprüche 1 bis 3 hergestellten tautomeren Form I als Lichtschutzmittel in kosmetischen Zubereitungen.

## Claims

1. A process for the preparation of the tautomeric form I of 2,4,6-trianilino-p-(carbo-2'-ethylhexyl-1'-oxy)-1,3,5-triazine 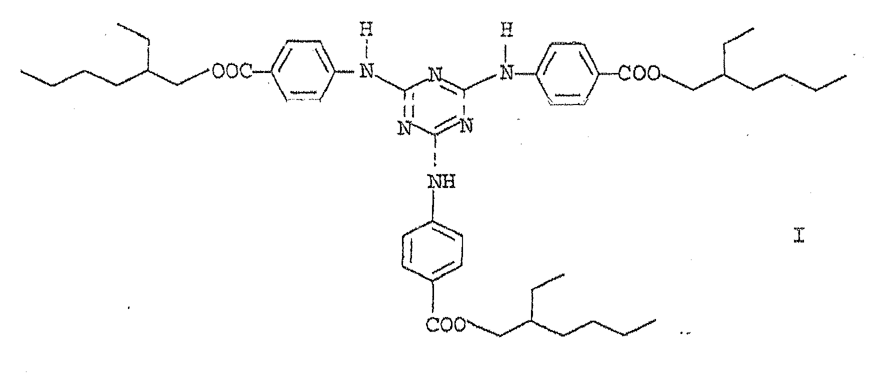 by crystallization from a tautomer mixture in the presence of one or more solvents chosen from the group consisting of aliphatic alcohols having 2 to 8 carbon atoms, aliphatic carboxylic alkyl esters having a total of 3 to 10 carbon atoms, aromatic carboxylic alkyl esters having a total of 8 to 12 carbon atoms, aliphatic carbonic esters having a total of 3 to 9 carbon atoms, carbonitriles having a total of 2 to 8 carbon atoms, dialkyl ketones having a total of 3 to 6 carbon atoms and aliphatic sulfones having a total of 3 to 6 carbon atoms, where the solvent or the solvent mixture may additionally comprise up to 30% by weight of a hydrocarbon.

2. The process according to claim 1, wherein the crystallization is carried out in an aliphatic alcohol having 2 to 4 carbon atoms, in an aliphatic carboxylic alkyl ester having a total of 3 to 6 carbon atoms or a mixture thereof.

3. The process according to either claim 1 or 2, wherein the crystallization is carried out in ethanol, ethyl acetate or a mixture thereof.

4. The use of the tautomeric form I prepared as in any of claims 1 to 3 as light protection agent in cosmetic preparations.

## Revendications

1. Procédé pour la préparation de la forme tautomère I de la 2,4,6-trianilino-p-(carbo-2'-éthylhexyl-1'-oxy)-1,3,5-triazine 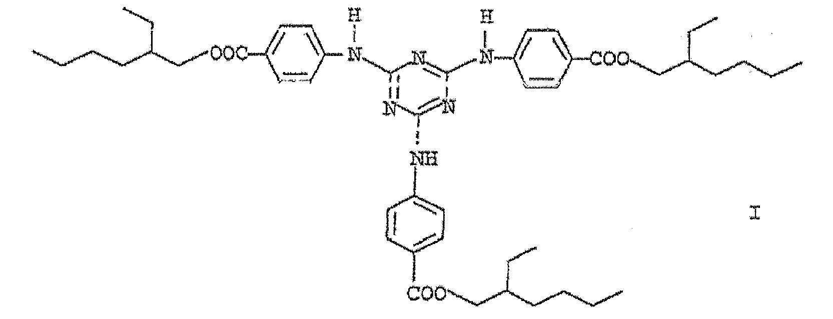 par cristallisation à partir d'un mélange de tautomères, en présence d'un ou de plusieurs solvants, choisis dans le groupe constitué par les alcools aliphatiques comprenant 2 à 8 atomes de carbone, les esters alkyliques d'acides carboxyliques aliphatiques comprenant au total 3 à 10 atomes de carbone, les esters alkyliques d'acides carboxyliques aromatiques comprenant au total 8 à 12 atomes de carbone, les esters aliphatiques de l'acide carbonique comprenant au total 3 à 9 atomes de carbone, les nitriles d'acides carboxyliques comprenant au total 2 à 8 atomes de carbone, les dialkylcétones comprenant au total 3 à 6 atomes de carbone et les sulfones aliphatiques comprenant au total 3 à 6 atomes de carbone, où le solvant ou le mélange de solvants peut en outre encore contenir jusqu'à 30% en poids d'un hydrocarbure.

2. Procédé selon la revendication 1, **caractérisé en ce qu'**on réalise la cristallisation dans un alcool aliphatique comprenant 2 à 4 atomes de carbone, dans un ester alkylique d'acide carboxylique aliphatique comprenant au total 3 à 6 atomes de carbone ou un mélange de ceux-ci.

3. Procédé selon l'une quelconque des revendications 1 ou 2, **caractérisé en ce qu'**on réalise la cristallisation dans l'éthanol, l'ester éthylique de l'acide acétique ou un mélange de ceux-ci.

4. Utilisation de la forme tautomère I préparée selon l'une quelconque des revendications 1 à 3 comme agent de protection contre la lumière dans des préparations cosmétiques.
